# EUROPÄISCHE PATENTSCHRIFT

(11) **EP 1 974 655 B1**
(45) Veröffentlichungstag und Bekanntmachung des Hinweises auf die Patenterteilung: **20.03.2013**
(21) Anmeldenummer: 08012683.2
(22) Anmeldetag: 28.08.2002
(51) Int. Cl.: A61B 3/10

(54) **Verfahren, Vorrichtung und Anordnung zur Messung des dynamischen Verhaltens eines optischen Systems**
Method, device and arrangement for measuring dynamic behaviour of an optical system
Procédé, dispositif et ensemble de mesure du comportement dynamique d'un système optique

(30) Priorität: 07.11.2001 DE 10154194
(43) Veröffentlichungstag der Anmeldung: 01.10.2008
(62) Teilanmeldung aus: 02774539.7
(73) Patentinhaber: Carl Zeiss Meditec AG, 07745 Jena (DE)
(72) Erfinder: Vogelsang, Hartmut, 07743 Jena (DE); Bergt, Michael, 07745 Jena (DE); Dick, Manfred, 07926 Gefell (DE); Mäusezahl, Holger, 07745 Jena (DE); Schröder, Eckhard, 90542 Eckenthal (DE)
(74) Vertreter: DTS München

(56) Entgegenhaltungen:
- WO-A-00/19885
- US-A- 6 155 684
- NAVARRO R ET AL: "PHASE PLATES FOR WAVE-ABERRATION COMPENSATION IN THE HUMAN EYE" OPTICS LETTERS, OPTICAL SOCIETY OF AMERICA, WASHINGTON, US, Bd. 25, Nr. 4, 15. Februar 2000 (2000-02-15), Seiten 236-238, XP000947124 ISSN: 0146-9592
- HUANG J ET AL: "Dynamic modeling and identification of an adaptive optics system" CONTROL APPLICATIONS, 1995., PROCEEDINGS OF THE 4TH IEEE CONFERENCE ON ALBANY, NY, USA 28-29 SEPT. 1995, NEW YORK, NY, USA,IEEE, US, 28. September 1995 (1995-09-28), Seiten 456-463, XP010207488 ISBN: 0-7803-2550-8
- HOFER H ET AL: "DYNAMICS OF THE EYE'S WAVE ABERRATION" JOURNAL OF THE OPTICAL SOCIETY OF AMERICA - A, OPTICAL SOCIETY OF AMERICA, WASHINGTON, US, Bd. 18, Nr. 3, März 2001 (2001-03), Seiten 497-506, XP001041247 ISSN: 1084-7529
- CHAO C ET AL: "Dynamic retinal image reconstruction of the human eye" ISSPA '99. PROCEEDINGS OF THE FIFTH INTERNATIONAL SYMPOSIUM ON SIGNAL PROCESSING AND ITS APPLICATIONS (IEEE CAT. NO.99EX359) QUEENSLAND UNIV. TECHNOL BRISBANE, QLD., AUSTRALIA, Bd. 2, 1999, Seiten 919-922 vol.2, XP002492454 ISBN: 1-86435-451-8

## Beschreibung

Die Erfindung betrifft ein Verfahren und eine Vorrichtung zur Messung des dynamischen Verhaltens eines optischen Systems.

Die Analyse optischer Wellenfronten von abbildenden Systemen und Lasersystemen hat eine zunehmende Bedeutung erlangt, da dies Ausgangspunkt für die Qualitätssteigerung dieser Systeme ist. Mit der Verfügbarkeit kommerzieller Shack-Hartmann-Sensoren (z. B. SCLA-series, WavefrontSciences, http://wavefrontsciences.com) kann man Aberrationen sehr genau erfassen und in Form von Zernike-Polynomen verschiedener Ordnungen oder alternativer Darstellungen klassifizieren.

Auch andere Aberrometer nach dem Tscherning-Prinzip, nach Abbe oder das Tracey-Aberrometer (ray-tracing) oder Systeme nach dem Skiaskop-Prinzip erlauben die Erfassung der höheren Aberrationen.

Systeme wie z. B. Shack-Hartmann-Sensoren, die mit einem CCD-Chip zur Speicherung der optisch relevanten Information ausgestattet sind, ermöglichen eine Datenakquirierung mit Videobildfrequenzen vorzunehmen und deshalb dynamische Abläufe ausreichend schnell aufzuzeichnen.

Bekannt sind Methoden, über die normale sphärische und zylindrische Korrektur der Abbildungsfehler hinausgehend, auch die höheren Aberrationen ab dritter Ordnung gemäß Seidel bzw. Zernike-Klassifizierung zu korrigieren. Dazu verwendet man z. B. adaptive Optiken, die als deformierbare Spiegel in Reflexion wirken oder Flüssigkristalloptiken, die in Transmission arbeiten. Diese adaptiven Optiken sind technologisch aufwendig und z. Z. noch nicht in allen Konsequenzen ausgereift. Sie erreichen z. Z. flächenhafte Auflösungen von typischerweise einigen Quadratmillimetern und werden bereits unter Laborbedingungen eingesetzt, um Wellenfronten in Rückkopplungsverfahren zwischen Wellenfrontmessung und adaptivem Element zu beeinflussen (siehe dazu Fernández, E. J. Iglesias, I., Artal, P. "Closed Loop Adaptive Optics in the Human Eye", Optics Letters, vol. 26, No. 10, May 15, 2001). Diese Systeme sind bisher nicht eingesetzt worden, um neben der reinen Korrektur von Aberrationen hinausgehend echte Dynamikstudien bei Variation verschiedenster Sehbedingungen vorzunehmen. Des Weiteren wurden insbesondere für ophtalmologische Anwendungen Methoden aufgezeigt, durch das optische System des Auges deformierte Wellenfronten auf einen Idealwert hin zu korrigieren, indem Aberrationen höherer Ordnung integral berücksichtigt werden (siehe dazu Optics Letters, Vol. 25 No. 4/February 15, 2000, 236 - 238, AWACS-Asclepion Wavefront Aberration Correction Simulator - Firmenunterlagen zur Präsentation auf der ESCRS in Brüssel, September 2000 und AAO in Dallas, Oktober 2000).

Ferner ist aus der Patentanmeldung WO 00/19885 A eine Vorrichtung zum Messen der Refraktion eines Auges bekannt. Die Vorrichtung weist eine Lichtquelle auf die einen Sondierungsstrahl entlang eines Pfades zum Auge erzeugt, ein teleskopisches System von Linsen, das eine Eintrittspupille und eine Austrittspupille aufweist, einen Zwei-Koordinaten-Ablenker, der aus zwei durch eine Zone getrennten Einzel-Koordinaten-Ablenkern besteht, eine Ablenkungs-Steuereinheit, eine Aperturblende, eine Feldblende, eine Sammellinse, einen positionsempfindlichen Fotodetektor mit einer Objektivlinse, die im Betrieb angeordnet ist, um aus der Retina des Auges reflektiertes Licht zu empfangen, sowie eine Datenverarbeitungs- und Anzeigeeinheit, die einen Rechner einschließt. Das teleskopische System ist im Sondierungsstrahlpfad nach dem Zwei-Koordinaten-Ablenker in einem Abstand entsprechend der Koinzidenz des Eintrittspupillen-Brennpunkts des teleskopischen Systems und der Zone zwischen den Einzel-Koordinaten-Ablenkern angeordnet. Die Aperturblende ist zwischen den Linsen des teleskopischen Systems am Koinzidenzpunkt ihrer Brennpunkte angeordnet und die Feldblende ist in der Ebene der Austrittspupille des teleskopischen Systems und am Positionierungspunkt des vorderen Brennpunkts der Sammellinse angeordnet.

Es ist daher Aufgabe der vorliegenden Erfindung, ein Verfahren und eine Vorrichtung bereitzustellen, mit denen das dynamische Verhalten eines optischen Systems objektiv erfassbar ist.

Dieses Problem wird durch ein Verfahren gemäß Anspruch 1 zur Messung des dynamischen Verhaltens eines optischen Systems gelöst, bei dem das zu messende optische System durch Reize zu einer Reaktion stimuliert wird und die Reaktion mittels einer Wellenfrontanalyse erfasst wird.

Das dynamische Verhalten des optischen Systems sind insbesondere Anpassungsvorgänge an veränderte Sehbedingungen, beispielsweise eine Akkomodation oder eine Blendenverstellung (Adaption). Das optische System kann ein Auge, beispielsweise ein menschliches Auge, ein künstliches Auge oder eine beliebige, künstliche Vorrichtung sein. Der Reiz kann grundsätzlich beliebiger Natur sein. Die Reaktion ist das dem Reiz nachfolgende dynamische Verhalten des optischen Systems. Die Wellenfrontanalyse kann beispielsweise mit einem Aberrometer, insbesondere mittels Shack-/Hartmann-Sensoren, aber auch mit Aberrometern nach dem Tscherning-Prinzip, nach Abbe, mit einem Ray-Tracing-Aberrometer oder System nach dem Siaskop-Prinzip durchgeführt werden. Bei der Akkomodation kann insbesondere der jeweils aktuelle Fokus des optischen Systems und dessen zeitlicher Verlauf erfasst werden. Ebenso können unterschiedliche Blendeneinstellungen bezüglich Ihres zeitlichen Verlaufs erfasst werden. Das dynamische Verhalten kann dabei beispielsweise auch unter Einfluß von Medikamenten untersucht werden.

In einer Ausgestaltung des Verfahrens ist vorgesehen, dass die Reize optische und/oder mechanische und/oder elektrische Reize sind. Veränderliche optische Reize können beispielsweise in Form von aktiven steuerbaren Lichtquellen, beleuchteten Darstellungen oder dergleichen bewirkt werden. Insbesondere kann der die Abbildungsschärfe und/oder der Objektabstand und/oder dessen Fokus und/oder die Intensität des optischen Reizes verändert werden, sodass eine Akkomodation bzw. eine Blendenverstellung (Adaption) des optischen Systems provoziert werden kann. Ebenso kann eine Aberration auch höherer Ordnung, z.B. in Form einer entsprechend "deformierten" Objektwellenfront als optischer Reiz verwendet werden. Veränderliche mechanische Reize können beispielsweise als Luftzug durch ein Gebläse erzeugt werden. Veränderliche chemische Reize sind z.B. durch Rauch oder das Einbringen einer Flüssigkeit, eines Gases oder eines Aerosols realisierbar. Veränderliche elektrische Reize sind unmittelbar durch an dem Auge oder im Bereich des Auges applizierte Elektroden oder durch induktive oder kapazitive Einkoppelung eines elektrischen Signals möglich. Die genannten Reize können jeweils einzeln oder beliebig miteinander kombinierbar aufgebracht werden und sowohl abrupt als auch kontinuierlich verändert werden.

In einer Ausgestaltung des Verfahrens ist vorgesehen, dass das zu messende optische System ein menschliches Auge ist. Das Verfahren zielt darauf ab, bei Stimulierung des einzelnen Auges oder des Augensystems beim Sehprozess eine gezielte Erregung und damit einhergehende Beeinflussung der Augenparameter herbeizuführen. Die mit den Beeinflussungen verbundenen Veränderungen der Augenparameter verändern unmittelbar die Abbildungseigenschaften des Auges und sind damit beispielsweise durch eine synchron zur Erregung getriggerte Wellenfrontmessung zugänglich. Mit Hilfe dieses Verfahrens können unterschiedlichste Effekte untersucht und gemessen werden. Beispielsweise kann die Zeitabhängigkeit und Geschwindigkeit der Akkommodation und der Akkommodationsfähigkeit, die Adaption und Adaptionsfähigkeit oder -geschwindigkeit des Auges unter Einflüssen wie z. B. Aberration, Beleuchtung, Medikamentation oder psychischer Einflüsse untersucht werden. Das dynamische Kurz- und Langzeitverhalten von Kontaktlinsen, beispielsweise Rutschen oder dergleichen, kann bezüglich der Veränderungen der Aberration durch Tragen von Kontaktlinsen untersucht werden. Das dynamische Verhalten von Intraokularlinsen (IOL), akkommodationsfähiger Intraokularlinsen und die Wechselwirkung des residualen Ziliarkörpers auf Intraokularlinsen sowie deren Passung und Bewegung und ggf. induzierter Akkommodation kann erfaßt werden. Des Weiteren sind Zusammenhänge zwischen dem physischen Sehen und der Gehirnleistung, die u. U. objektive Rückschlüsse auf Krankheitsbilder wie Kopfschmerzen durch Überanstrengung aufdecken helfen können, möglich. So können beispielsweise Blendwirkungen und andere zeitlich veränderliche Eindrücke im Zusammenhang mit Ermüdungserscheinungen beim Autofahren gemessen werden. Die optische dynamische Korrektur kann beispielsweise berufsgruppenbezogen, also auf bestimmte Anforderungen an das Sehvermögen, untersucht werden.

In einer Ausgestaltung des Verfahrens ist vorgesehen, dass die Stimulation des zu messenden Auges mit dem Aberrometer synchronisiert wird. Auf diese Weise ist es möglich, die gemessenen Adaptionsvorgänge des Auges unmittelbar dem jeweiligen Reiz und dessen zeitlichem Verlauf zuzuordnen. Die Synchronisierung kann dabei z.B. zeitlich oder bezüglich der Intensität zwischen dem jeweiligen Reiz und der Messung der Aberrometrie erfolgen

Bei der Durchführung des Verfahrens kann eines oder beide menschlichen Augen zu einer Reaktion stimuliert werden, ebenso kann eines oder beide menschlichen Augen gemessen werden. Aufgrund der gewonnenen stimulierten, dynamischen Messergebnisse können für das jeweils gemessene spezielle Auge optimierte/gemittelte Werte zur statischen/stationären Korrektur der Wellenfront abgeleitet werden. Damit kann man dem Auge für sein spezielles Aktionsspektrum eine optimale Wellenfrontkorrektur bereitstellen. Außerdem kann man für spezielle Sehvorgänge maßgeschneiderte Lösungen bereitstellen. Als Beispiele seien das Nachtsehen, geschwindigkeitsoptimierte Akkomodation oder das Nah- bzw. Fernsehen genannt.

Das eingangs genannte Problem wird auch durch eine Vorrichtung gemäß Anspruch 5 gelöst, die eine Stimulationseinheit und ein Aberrometer umfasst. Mit der Stimulationseinheit können gezielt Anpassungsvorgänge auf äußere Reize des zu untersuchenden optischen Systems ausgelöst werden. Die Stimulationseinheit ist dazu so ausgelegt, dass diese äußere Reize auf das optische System ausüben kann. Als äußere Reize kommen prinzipiell alle physikalischen oder chemischen Effekte oder Mittel in Betracht, die eine Anpassungsreaktion des optischen Systems hervorrufen. Die Stimulationseinheit kann abrupt und/oder kontinuierlich auf das optische System einwirken und die entweder vor dem ungemessenen optischen System oder dem zu messenden optischen System angeordnet sein. Als Aberrometer werden hier allgemein Vorrichtungen zur Wellenfrontmessung oder zur Messung der Aberration verstanden. Dies können sowohl Vorrichtungen mit elektronischer Datenerfassung als auch manuell bedienbare Vorrichtungen sein.

Eine besonders einfache und zudem automatisierbare Auswertung der optischen Daten wird ermöglicht, da das Aberrometer eine Vorrichtung zur Wellenfrontanalyse umfasst. Die Vorrichtung zur Wellenfrontanalyse kann z. B. ein Shack-Hartmann-Sensor sein, der mit einem CCD-Chip zur Speicherung der optisch relevanten Information ausgestattet ist. Die Messergebnisse und damit das Ergebnis des dynamischen Anpassungsvorganges können mittels Datenübergabe in eine Softwareanwendung grafisch visualisiert werden.

Besonders vorteilhaft ist es, wenn die Stimulationseinheit einen optischen und/oder mechanischen und/oder elektrischen und/oder chemischen Reiz auslösen kann. Insbesondere kann ein optischer Reiz so ausgelegt sein, dass eine Akkomodation oder Blendenvorstellung des optischen Systems provoziert wird. Auf diese Weise können unterschiedliche Reize auf das Auge ausgeübt werden und so auch Alltagssituationen wie z. B. Zugluft oder durch Rauch ausgelöste chemische Reize oder dergleichen simuliert werden.

Die Stimulationseinheit kann vor dem an dem Aberrometer vorbei schauenden Auge angeordnet sein, alternativ kann z.B. der Strahlengang der Stimulationseinheit in das Aberrometer eingespiegelt sein. Bei der erst genannten Ausführungsform der Vorrichtung kann das nicht zu untersuchende Auge durch Reize stimuliert werden, bei der zweit-genannten Ausführungsform der Vorrichtung kann das zu untersuchende Auge direkt durch äußere Reize stimuliert werden.

Alternativ kann die Stimulationseinheit in das Aberrometer integriert sein. In diesem Fall wird in einem gemeinsamen Gehäuse der Strahlengang der Stimulationseinheit unmittelbar in den des Aberrometers eingekoppelt. Dies ermöglicht eine sehr kompakte Bauweise.

Die Vorrichtung kann sowohl für monokulares als auch für binokulares Sehen ausgeführt sein. Ebenso kann die Stimulation ein oder beide Augen betreffen. Es können daher beliebige Kombinationen der Messung eines Auges und der Stimulation eines Auges realisiert sein, beispielsweise Stimulation eines Auges und Messung des anderen Auges, Stimulation eines Auges und Messung des gleichen Auges, Stimulation eines Auges und Messung beider Augen, Stimulation beider Augen und Messung eines Auges oder beider Augen.

In einer bevorzugten Ausführungsform ist vorgesehen, dass die Stimulationseinheit ein Fixierungsobjekt umfasst. Dieses soll von dem Probanden fixiert werden, so dass eine definierte Fokussierung des Auges erreicht wird. Das Fixierungsobjekt ist eine von dem Probanden leicht erkennbare bildliche Darstellung oder ein definierter Lichtfleck. Bevorzugt kommt hier ein fein strukturiertes Bild oder eine aus mehreren Elementen, die beispielsweise farblich abgestuft sein können, zusammengesetzte Lichtquelle in Betracht.

Vorteilhaft ist es, wenn das Fixierungsobjekt einen Lichtreiz abgeben kann, der in seiner Intensität und/oder Fokussierung veränderbar ist. Das Auge, das das Fixierungsobjekt fokussiert, kann auf diese Weise unmittelbar stimuliert werden. Die Änderung der Intensität kann beispielsweise durch eine Veränderung der Leuchtstärke bei Lichtquellen als aktiven Elementen oder durch Veränderung der Beleuchtungsstärke bei passiven Elementen wie einer beleuchteten Graphik erreicht werden. Eine Änderung der Fokussierung ist beispielsweise durch das Verfahren des Fixierungsobjekt selbst oder durch eine Veränderung vorgeschalteter Linsen möglich.

Das Fixierungsobjekt ist bevorzugt eine beleuchtete Grafik. Es handelt sich dabei um ein Objekt, das von dem Probanden leicht und sicher erkannt und fixiert werden kann und zudem einfach zu realisieren ist.

Die Vorrichtung kann mindestens eine in den Strahlengang der Stimulationseinheit und/oder des Aberrometers einbringbare Phasenplatte umfassen. Vorteilhaft wird ein Satz von Phasenplatten verwendet, welche nach den unterschiedlichen Zernike-Polynomen mit abgestuften Amplituden sortiert sind und die ähnlich Probierlinsen beim bekannten Phoropter vor dem zu untersuchenden System positioniert werden können. Als Phasenplatten können zum Beispiel transparente Glas- oder Kunststoffplatten benutzt werden, deren Oberfläche so strukturiert ist, dass einer durchlaufenden Lichtwelle definierte Aberrationen beispielsweise einem einzelnen Zernike-Term entsprechend aufgeprägt werden. In einer Wechselvorrichtung, vorzugsweise einem Wechselrevolver, sind vorzugsweise Platten einer Ordnung der Zernike-Koeffizienten mit unterschiedlicher Amplitude angeordnet. Durch zentrierte Anordnungen mehrerer solcher Wechselvorrichtungen hintereinander ist es möglich, unterschiedliche Phasenplatten in die optische Sehachse des zu untersuchenden optischen Systems einzuschwenken. Auf diese Weise kann auf der optischen Sehachse des zu untersuchenden, optischen Systems eine fein abstufbare Kombination von Abbildungsfehlern höherer Ordnung eingebracht werden. Mit dieser Anordnung können insbesondere Sehfehler, die auf höherer Aberration beruhen, subjektiv nach Erreichen eines angepassten, stationären Zustandes beispielsweise des menschlichen Auges bewertet werden. Die Bewertung erfolgt beispielsweise mit einer Zeitskala, z.B. nach einigen Sekunden der Anpassung.

Die Vorrichtung kann zur Auswertung der unterschiedlich stimulierten dynamischen Messdaten des Auges eine Software enthalten, die einen optimalen oder gemittelten Wert der Wellenfront bereitstellt, der zur statischen/stationären Korrektur der optischen Sehfehler bei den bekannten Korrekturverfahren (Brille, CL, IOL, LASIK, PRK, ...) verwendet wird.

Es kann auch eine Trennung der zuvor im Rahmen der Vorrichtung gemeinsam realisierten Elemente vorgenommen werden. Es kann daher auch eine herkömmliche Vorrichtung zur Messung der Aberration eines Auges mit einer eigenständigen Stimulationseinheit verwendet werden.

Vorteilhafte Ausgestaltungen der Erfindung werden weiter in den Zeichnungen erläutert. Dabei zeigen:
- Fig. 1: eine Prinzipskizze einer Vorrichtung zur Durchführung des Verfahrens;
- Fig. 2: eine erste Ausführungsform der erfindungsgemäßen Vorrichtung als Prinzipskizze;
- Fig. 3: eine zweite Ausführungsform der erfindungsgemäßen Vorrichtung als Prinzipskizze.

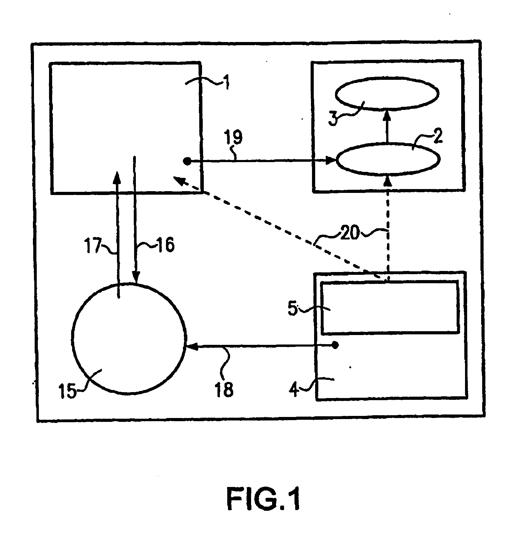

Zunächst wird auf Fig. 1 Bezug genommen. Diese zeigt die Prinzipskizze einer Vorrichtung sowie des damit zusammenhängenden Verfahrensablaufes zur dynamischen Stimulation und dynamischen Messung der Aberrometrie eines Auges 15. Dargestellt sind jeweils die einzelnen Komponenten in ihrem Wirkungszusammenhang. Die Vorrichtung umfasst ein Gerät zur Wellenfrontmessung/Aberrometrie des Auges 15, bei dem entweder beide Augen gleichzeitig oder jeweils ein Auge gemessen werden kann. Im letzten genannten Fall kann das gerade nicht gemessene Auge gegebenenfalls frei am Gerät vorbei blicken oder blickt ebenfalls in das Gerät. Der Einfachheit halber wird das Gerät zur Wellenfrontmessung/Aberrometrie eines Auges im Folgenden Aberrometer 1 genannt. Von dem Aberrometer 1 wird Messlicht 16 in das Auge 15 eingestrahhlt, welches Signallicht 17 zurückstrahlt. Messlicht 16 und Signallicht 17 sind durch Pfeile in Fig. 1 angedeutet. Die Vorrichtung umfasst des Weiteren eine Einrichtung zur dynamischen Datenakquirierung 2, die Rohdaten 19 des Aberrometers 1 entgegennimmt. Es kann sich dabei um gängige elektronische Vorrichtungen zur Messwerterfassung handeln. Diese Vorrichtung kann beispielsweise ein programmgesteuerter Rechner mit einer Software sein, die insbesondere sequenzielle Mess-Serien in Echtzeit akquirieren kann. Gegebenenfalls ist dafür eine Datenzwischenspeicherung in einem flüchtigen oder nicht flüchtigen Speicher z. B. eines Steuerungscomputers notwendig. Die Einrichtung umfasst des weiteren eine Analysesoftware, welche die akquirierten Daten bzw. Datensätze analysieren kann, um die bestimmenden Parameter der Wellenfront (z. B. Zernike-, Taylor-Koeffizienten) zu berechnen. Die Messergebnisse und damit das Ergebnis des dynamischen Anpassungsvorganges können mittels einer Softwareanwendung grafisch visualisiert werden. Dazu dient ein Analysemodul 3, an das die ermittelten Daten übergeben werden. Die Fähigkeiten der dynamischen Datenakquirierung 2 sowie des Analysemodules 3 können so kombiniert werden, dass eine der Hardware angepasste und gegebenenfalls in der Akquirierungsrate reduzierte sequenzielle Echtzeitmessung mit simultaner Analyse stattfinden kann. Des Weiteren ist eine Stimulationseinheit 4 zur abrupten und/oder kontinuierlich veränderlichen optischen Einwirkung wie beispielsweise Aberration, Lichteinfluss, Objektabstand oder dergleichen vorhanden, die entweder vor dem ungemessenen Auge 15 oder dem zu messenden Auge 15 angeordnet ist und die mit der dynamischen Datenakquirierung 2 synchronisiert sein kann. Die Stimulation des Auges 15 ist in Fig. 1 durch einen Pfeil 18 angedeutet Eine Synchronisierungseinheit 5 dient der Synchronisierung zwischen Stimulationseinheit 4 und Aberrometer 1. Die Synchronisierungseinheit kann Synchronisierungsimpulse 20, in Fig. 1 durch gestrichelte Pfeile angedeutet, an das Aberrometer und/oder die Einrichtung zur dynamischen Datenaquirierung 2 abgeben.

Die Stimulation des Auges kann auch über Sehtafeln und dergleichen erfolgen, wobei eine Synchronisierung nicht vorzuliegen braucht. Damit entfällt dann die Verbindung zwischen der Stimulationseinheit 4 und dem Aberrometer 1 und/oder der dynamischen Datenakquirierung 2. Aberrometer 1, dynamische Datenakquirierung 2, Analysemodul 3 sowie Stimulationseinheit 4 können auch als Einheit und damit einteilige Baugruppe realisiert sein.

Die Stimulationseinheit 4 kann auch als eigenständiges, separates Gerät realisiert sein und eingesetzt werden. Das dann vorliegende Gerät stellt eine Art Stimulationsphoropter dar, durch den der Proband z. B. eine Phasenplattenkorrektur mit oder ohne Variation anderer Sehparameter beurteilen kann. Die Synchronisierungseinheit 5 kann dann entfallen.

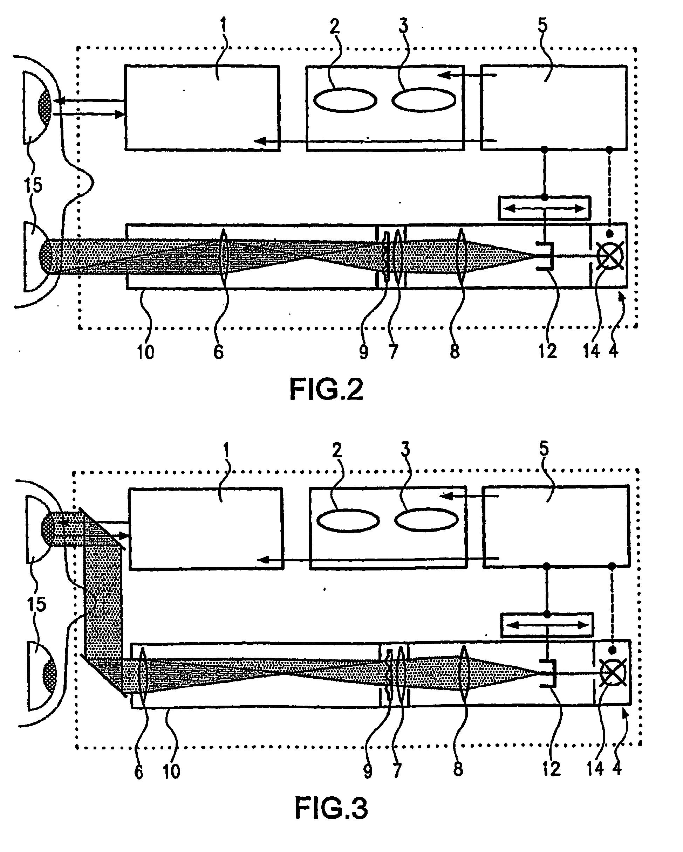

Die Fig. 2 und 3 zeigen Ausführungsformen der Vorrichtung. Die dargestellten Strahlverläufe durch eine erste Linse 6, eine zweite Linse 7 und eine dritte Linse 8 entsprechen nicht genau den realen Strahlverläufen bei Betrachtung im Rahmen der geometrischen Optik, sondern sollen nur zur Veranschaulichung dienen. Die konkrete Realisierung des optischen Konzeptes kann so erfolgen, dass die Nase des Patienten kein Hindernis darstellt. In den Fig. 2 und 3 ist der optische Aufbau so dargestellt, dass sich eine Phasenplatte 9 in einer zur Hornhautoberfläche oder Brillenkorrekturfläche konjugierten Ebene befindet. Durch Integration eines nicht dargestellten Mechanismus zum automatischen Wechsel der Phasenplatten 9, beispielsweise in Form eines Phasenplattenwechselrades oder dergleichen, können verschiedene Aberrationen bequem auch während oder zwischen Mess-Serien vorgenommen werden. Eine erste Blende 10 sowie eine zweite Blende 11 geben zusammen mit der Fixierung auf ein Fixierungsobjekt 12 die optische Achse vor. Gegebenenfalls können weitere Zieleinrichtungen vorgesehen werden, z.B. Flächenkreuze. Die Zentrierung kann darüber hinaus durch ein Videobild kontrolliert werden, das innerhalb der Vorrichtung der Fig. 2 und 3 beispielsweise durch Strahlteiler abgegriffen werden kann. Variierende Akkommodationszustände können durch Verfahren des Fixierungsobjektes entlang eines Verfahrweges 13 oder alternativ beispielsweise der dritten Linse 8 stimuliert werden. Die Adaption kann durch Einstellung der Beleuchtung des Fixierungsobjektes 12 durch eine Lichtquelle 14 beeinflusst werden. Zusätzlich oder alternativ kann eine direkte Einstellung der Helligkeit des Raumlichtes und/oder des Umgebungslichtes stattfinden. Diese Möglichkeit ist der Einfachheit halber nicht dargestellt. Die einzelnen Komponenten können elektrisch bzw. elektromotorisch angesteuert und angetrieben werden. Die Synchronisierung dieser Komponenten mit der Messung und Datenakquirierung kann dann z. B. bequem über die Abfrage programmierbarer Schnittstellen erfolgen.

Bei der Vorrichtung nach Fig. 2 wird ein Auge 15 gemessen und das jeweils andere Auge 15 stimuliert, bei der Vorrichtung nach Fig. 3 wird das zu messende Auge 15 gleichzeitig stimuliert. Zunächst wird der gemeinsame Aufbau in der Fig. 2 erläutert. Die Vorrichtung umfasst ein Aberrometer 1, es handelt sich dabei um ein Gerät zur Messung der Wellenfrontverformung durch das optische System des Auges und damit zur Bestimmung und Klassifizierung der Abbildungsfehler des Auges 15, dabei werden auch Abbildungsfehler höherer Ordnung umfasst. Das Aberrometer 1 ist gekoppelt mit einer Einrichtung zur dynamischen Datenakquirierung 2. Es handelt sich hier um ein Modul zur Ansteuerung des Aberrometers 1. Dieses Modul kann einen Wellenfrontmessvorgang auslösen. Ebenso können die gewonnenen Messdaten zwischengespeichert werden und aus den Messdaten sodann die gemessene Wellenfront rekonstruiert werden. Dabei können beispielsweise die Rohdaten des Videobildes des Sensors oder auch vollständig ausgewertete Wellenfrontparameter wie beispielsweise Zernike-Koeffizienten gespeichert werden. Messung und eventuelle Auswertung und Speicherung erfolgen mit Taktraten, die schneller als der zu untersuchende Anpassungsvorgang sind. Die Taktraten können beispielsweise im Bereich von 10 bis 100 Hz liegen.

Die Einrichtung zur dynamischen Datenakquirierung 2 ist mit einem Analysemodul 3 gekoppelt. Das Analysemodul 3 dient der Auswertung der Sensordaten und gegebenenfalls der Rekonstruktion und grafischen Visualisierung der gemessenen und gespeicherten Wellenfronten und ist weitgehend in Software realisierbar. Des Weiteren kann eine Parametrisierung der Wellenfront z. B. durch Entwicklung nach Zernike-Polynomen oder durch zonale Rekonstruktion durchgeführt werden. Der Analyseprozess kann in enger Kopplung mit der Einrichtung zur dynamischen Datenakquirierung 2 gekoppelt sein und den Analyseprozess teilweise oder vollständig vor der Zwischenspeicherung vornehmen.

Eine Stimulationseinheit 4 besteht im Wesentlichen aus einem optischen System, das dem zu untersuchenden oder dem freien Auge 15 ein zu fixierendes, detailliert strukturiertes Sehobjekt darbietet und das Auge 15 zur Fokussierung auf die Struktur des Objektes bewegt. Die Stimulationseinheit 4 kann optische Elemente wie z. B. Linsen oder Phasenplatten aufweisen, die die vom Sehobjekt ausgehende Wellenfront verformen, bevor sie ins Auge 15 gelangen. Dadurch kann das Auge 15, das bemüht ist, ein scharfes Bild vom Sehobjekt zu erhalten, zu Anpassungsreaktionen innerhalb des optischen Systems stimuliert werden. Bevorzugt können die abbildenden Eigenschaften der Stimulationseinheit 4 zeitlich variiert werden, um eine dynamische Reaktion des Auges 15 hervorzurufen. Die Stimulationseinheit 4 kann Informationen über Ihren momentanen Zustand an eine Synchronisationseinheit 5 mit der Wellenfrontmessung senden.

Die Synchronisationseinheit 5 ist ein Modul zur Synchronisation von dynamischen Veränderungen in der Stimulationseinheit 4 mit der Einrichtung zur dynamischen Datenakquirierung 2. Ziel ist es, die gemessenen Wellenfrontdaten mit den jeweiligen Zuständen der Synchronisationseinheit zu korrelieren.

Die in den Fig. 2 und 3 dargestellten Lösungen eines Aberrometers 1 können als dynamische Stimulations-Aberroskope bezeichnet werden. Hier ist zu beachten, dass das optische Konzept so vorgenommen werden kann, dass das ungemessene Auge nicht wie dargestellt in seinem Blick eingeschränkt werden muss, sondern frei blicken kann. Als vereinfachte Variante hierzu könnte die Synchronisierung zwischen der Stimulationseinheit 4 und Aberrometer 1 sowie der Einrichtung zur dynamischen Datenakquirierung 2 entfallen. Weitere Varianten benutzen zur Stimulation statt eines integrierten Fixierungsobjektes z. B. eine ruhende oder zur Abstandsvariation bewegliche Sehtafel und/oder einen Phasenplattenphoropter.

Die in Fig. 2 gezeigte Ausführungsform für eine Stimulationseinheit 4 ist auch als Verbesserung zum einfachen Phasenplattenphoropter zu sehen und kann in den verschiedenen Varianten z. B. mit einer Korrektur der Aberration und einer einfachen Sehtafel als Fixierungsobjekt usw. als alleinstehendes Gerät ausgelegt werden.

Für die gezielte Aufprägung von Aberrationen auf die Objektwellenfront kann wahlweise statt einer Phasenplatte und/oder der abbildenden Optik gemäß Fig. 2 und 3 auch ein adaptives optisches Element eingebracht werden. Die dabei stimulierten dynamischen Veränderungen der Abbildungseigenschaften des Auges 15 werden mit dem Aberrometer 1 in einer zeitlichen Sequenz dynamisch erfasst, die mit der Variation der Abbildungseigenschaften durch die adaptive Optik synchronisiert werden kann. Transmissionsbasierte adaptive Elemente wie z. B. Flüssigkristall-Phasenmodulatoren können z. B. statt der Phasenplatte in ähnlicher Weise in die Anordnung gemäß den Fig. 2 und 3 eingebaut werden.

Obwohl die Verwendung einer adaptiven Optik aufwendiger ist, da zusätzlich eine elektronische Ansteuerung der adaptiven Optik erforderlich wird, bieten sich Möglichkeiten der Stimulation, die Geräte mit Phasenplatten nicht oder nur schwer erlauben. Je nach Ansteuergeschwindigkeit der adaptiven optischen Elemente können beliebige Aberrationen der Objektwellenfront dynamisch verändert oder gezielt appliziert werden.

Die Stimulationseinheit 4 wird als optisches System ausgelegt, welches entweder vor dem frei vorbei schauenden Auge 15 platziert wird oder in den Strahlengang des mit dem Aberrometer untersuchten Auges eingespiegelt wird, wie dies in Fig. 3 dargestellt ist, oder die Stimulationseinheit 4 wird in das Aberrometer 1 integriert. Auch bei dieser letzten Ausführungsform sind wiederum zwei Varianten möglich, die Stimulationeinheit kann auf das oder die gemessenen Augen 15 einwirken oder die Stimulationseinheit 4 kann auf das ungemessene Auge 15 einwirken.

Bei der Stimulationseinheit selbst handelt es sich um ein optisches Gerät, bei dem ein Fixierungsobjekt vor das oder die gemessenen oder ungemessenen bzw. frei blickenden Augen platziert wird und auf dessen Zentrum das jeweilige Auge während der Untersuchung blicken und fokussieren soll. Die optische Wirkung bei der Stimulation kann entweder abrupt oder kontinuierlich moduliert werden, was z. B. durch Änderung der Entfernung, Leuchtdichte usw. des Fixierungsobjektes erreichbar ist und/oder durch Anbringen von Phasenplatten. Alle optisch wirksamen Modifikationen sind beliebig kombinierbar. Wird die optische Wirkung mit der dynamischen Messung des Auges 15 durch das Aberrometer 1 synchronisiert z. B. im Sinne einer Zeitsynchronisierung oder einer Beleuchtungssynchronisierung, so erhält man Ausführungsformen der Geräte entsprechend der Fig. 2 und 3. Die Wirkung muss nicht zwangsläufig synchronisiert werden. Informationen über die dynamischen Reaktionen des Auges bzw. der Augen können auch unsynchronisiert gemessen werden. Dazu kann z. B. über die Kenntnis der Datenakquirierungsfrequenz der Einrichtung zur dynamischen Datenakquirierung 2 eine zeitliche Zuordnung der Messwerte vorgenommen werden, diese können beispielsweise bei einer elektronischen Speicherung auf einem Datenträger oder dergleichen mit einem Zeitstempel versehen werden. Die Einrichtung zur dynamischen Datenakquirierung 2 und das Analysemodul 3 können auch so kombiniert werden, dass nicht nur eine dynamische Datenakquirierung, sondern gleichzeitig eine Hochgeschwindigkeitsanalyse der Daten erfolgt.

Das Fixierungsobjekt 12 kann z. B. durch eine beleuchtete Grafik mit hinreichend feiner Strukturierung realisiert werden, kann aber auch eine einfache, separat positionierte Sehtafel sein.

Die Abbildung des Fixierungsobjektes 12 kann durch Einschwenken von optischen Elementen wie Linsen erreicht werden und ggf. über ein Videosystem kontrolliert werden. Die Zentrierung auf eine vorgegebene Sehrichtung kann durch Blendensysteme optimiert werden. Eine automatische Positionierung z. B. erlaubt durch Variation von Abständen zwischen den optischen Komponenten und/oder dem Fixierungsobjekt gezielt Akkommodationszustände hervorzurufen. Eine geeignete Beleuchtungseinstellung des Fixierungsobjektes und/oder des Raumlichtes führt zu definierten Adaptionseinstellungen, die ebenfalls variabel gehalten werden können und zusätzliche Messparameter bilden.

Durch Einbringen speziell präparierter Phasenplatten mit definierter Oberflächentopografie in den Strahlengang der Stimulationseinheit 4 und/oder des Aberrometers 1 erlaubt die Vorrichtung die gezielte Applizierung von Aberrationen auf das oder die Augen 15. Die Phasenplatten können dazu in einem hier nicht dargestellten Wechselrevolver angeordnet sein und einzeln oder in Kombination in den Strahlengang der Stimulationseinheit 4 und/oder des Aberrometers 1 einbringbar sein.

Die zuvor dargestellte Vorrichtung und das damit ausführbare Verfahren dient der gezielten visuellen Stimulation eines biologischen oder künstlichen Auges 15 und zur Erfassung des damit verbundenen dynamischen Anpassungsvorgang des optischen Sehapparates durch Messen der Wellenfrontaberration. Die visuelle Stimulation, welche beim Blick in bzw. durch eine geeignete Apparatur hervorgerufen wird, führt zu einer Beeinflussung der Abbildungseigenschaften des Auges 15, welche gleichzeitig zeitsynchronisiert zur Stimulation in Echtzeit mit einem Wellenfrontanalysesystem bzw. einem Aberrometer 1 gemessen werden kann. Dies erlaubt völlig neue Diagnosemöglichkeiten, welche die Dynamik von Anpassungsvorgängen des Auges 15 zugänglich macht. Dazu werden Sehbedingungen wie Objektabstand und Helligkeit modifiziert und insbesondere gleichzeitig bestimmte Aberrationen gezielt korrigiert und/oder eingebracht. Damit kann z. B. der Einfluss bestimmter Aberrationsterme auf die Akkommodationsfähigkeit studiert werden, oder es kann untersucht werden, ob eine implantierbare Intraokularlinse vermöge des residualen Ziliarkörpers akkommodationsfähig ist und wie das gegebenenfalls optimal nutzbar wäre. Ein Vorteil der Erfindung liegt auch darin, subjektive Eindrücke bei der Bewertung von dynamischen Sehprozessen durch einen Probanden mit physikalisch objektiven Messdaten korrelieren zu können.

Mit der Vorrichtung und dem Verfahren ist es möglich, dynamische Änderungen der Abbildungseigenschaften des Auges zu stimulieren und deren zeitlichen Verlauf aufzuzeichnen. Mit Hilfe eines Systems von optischen Elementen können bestehende Abbildungsfehler auch höherer Ordnung zielgerichtet kompensiert und andere Abbildungsfehler gezielt zur Stimulation eingebracht werden, um die Einflüsse auf die Dynamik des optischen Systems des Auges 15 zu erfassen. Damit kann man verschiedenste dynamische Vorgänge wie z. B. während der Akkommodation oder Adaption in einer Bildfolge oder einem Film der Entwicklung der Wellenfrontaberrationen dokumentieren. Daraus lassen sich dynamische Parameter wie z. B. Anpassungsbereich, -zeiten, -geschwindigkeiten oder - beschleunigungen z. B. bei der Akkommodation oder der Adaption ableiten. Damit werden Rückschlüsse auf anatomische Parameter wie Elastizität der Augenlinse, die z. B. mit Fragestellungen der Wechselwirkung der Verformung von Augenlinse und Hornhaut und der Dynamik von Intraokularlinsen verbunden sein kann, oder auch das primäre Reaktionsvermögen des Auges objektiv messbar. Es können Grundzusammenhänge der Wirkung von Medikamentierung oder z.B. Ursache-Wirkungszusammenhänge von Krankheitsbildern wie z.B. Kopfschmerzen, Müdigkeit, Überanstrengung aufgedeckt werden.

Die beschriebene Erfindung bietet die Möglichkeit der objektiven dynamischen Messung der Abbildungseigenschaften des Auges bei gezielt stimulierten Anpassungsvorgängen unter vorgegebenen Randbedingungen.

### Bezugszeichenliste

- 1: Aberrometer
- 2: Einrichtung zur dynamischen Datenaquirierung
- 3: Analysemodul
- 4: Stimulationseinheit
- 5: Synchronisierungseinheit
- 6: Erste Linse
- 7: Zweite Linse
- 8: Dritte Linse
- 9: Phasenplatte
- 10: Erste Blende
- 11: Zweite Blende
- 12: Fixierungsobjekt
- 13: Verfahrweg
- 14: Lichtquelle
- 15: Auge
- 16: Messlicht
- 17: Signallicht
- 18: Stimulation
- 19: Rohdaten
- 20: Synchronisierungsimpulse

## Patentansprüche

1. Verfahren zur Messung des dynamischen Verhaltens eines optischen Systems umfassend die folgenden Schritte:
Stimulation eines zu messenden optischen Systems durch optische, mechanische oder elektrische Reize,
Erfassen der Reaktion des optischen Systems auf die Reize mittels einer Wellenfrontanalyse, wobei Wellenfrontaberrationen gemessen werden,
Ableiten von dynamischen Parametern bei der Reaktion des optischen Systems aus den Wellenfrontaberrationen,
**dadurch gekennzeichnet, dass** weiterhin aus den dynamischen Parametern Werte zur statischen oder stationären Korrektur einer Wellenfront des optischen Systems abgeleitet werden.

2. Verfahren nach Anspruch 1,
**dadurch gekennzeichnet, dass**
die dynamischen Parameter Anpassungsbereiche, Anpassungszeiten, Anpassungsgeschwindigkeiten oder Anpassungsbeschleunigungen bei der Akkommodation oder der Adaption umfassen.

3. Verfahren nach einem der Ansprüche 1 oder 2,
**dadurch gekennzeichnet, dass**
die dynamischen Parameter mittels Datenübergabe in einer Softwareanwendung grafisch visualisiert werden.

4. Verfahren nach einem der Ansprüche 1 bis 3, wobei die Reize stimulierte dynamische Veränderungen von Abbildungseigenschaften des optischen Systems provozieren,
**dadurch gekennzeichnet, dass**
diese Veränderungen von Abbildungseigenschaften mit einem Aberrometer (1) in einer zeitlichen Sequenz dynamisch erfasst werden.

5. Vorrichtung zur Messung des dynamischen Verhaltens eines menschlichen oder künstlichen Auges, umfassend eine Stimulationseinheit (4), welche Reize auf das Auge ausüben kann, ein Aberrometer (1), wobei das Aberrometer eine Vorrichtung zur Wellenfrontanalyse (3) umfasst, eine Einrichtung zur dynamischen Datenakquirierung (2) zur Entgegennahme von Rohdaten des Aberrometers, **gekennzeichnet durch** eine Synchronisationseinheit (5) zur Synchronisierung zwischen der Stimulationseinheit und dem Aberrometer.

## Claims

1. A method of measuring the dynamic behavior of an optical system, comprising the following steps:
stimulating an optical system to be measured by optical, mechanical or electrical stimuli,
detecting the reaction of the optical system to the stimuli by means of a wave front analysis in which wave front aberrations are measured,
deriving dynamic parameters with the reaction of the optical system from the wave front aberrations,
**characterized in that** values for a static or stationary correction of a wave front of the optical system are further derived from the dynamic parameters.

2. The method according to claim 1,
**characterized in that**
the dynamic parameters comprise adaptation areas, adaptation times, adaptation velocities or adaptation accelerations during the accommodation or adaption.

3. The method according to any of the claims 1 or 2,
**characterized in that**
the dynamic parameters are graphically visualized by means of data transfer in a software application.

4. The method according to any of the claims 1 to 3, in which the stimuli provoke stimulated dynamic alterations of imaging characteristics of the optical system,
**characterized in that**
these alterations of imaging characteristics are dynamically detected with an aberrometer (1) in a temporal sequence.

5. A device for measuring the dynamic behavior of a human or artificial eye, comprising a stimulation unit (4) capable of exerting stimuli on the eye, an aberrometer (1) comprising a wave front analyzing device (3), an equipment (2) for dynamically acquiring data for receiving raw data of the aberrometer, **characterized by** a synchronization unit (5) for synchronizing between the stimulation unit and the aberrometer.

## Revendications

1. Procédé de mesure du comportement dynamique d'un système optique comprenant les étapes suivantes :
stimulation d'un système optique à mesurer par excitation optique, mécanique ou électrique ;
détection de la réaction du système optique après l'excitation à l'aide d'une analyse de front d'ondes, avec mesure des aberrations de front d'ondes ;
déduction des paramètres dynamiques lors de la réaction du système optique à partir des aberrations de front d'ondes ;
**caractérisé en ce qu'**on déduit, en sus des paramètres dynamiques, des valeurs de correction statique ou stationnaire d'un front d'ondes du système optique.

2. Procédé selon la revendication 1, **caractérisé en ce que** les paramètres dynamiques comprennent des zones d'adaptation, des temps d'adaptation, des vitesses d'adaptation ou des accélérations d'adaptation lors de l'accommodation ou de l'adaptation.

3. Procédé selon l'une quelconque des revendications 1 ou 2, **caractérisé en ce que** les paramètres dynamiques sont visualisés sur le plan graphique par transmission de données dans une application logicielle.

4. Procédé selon l'une quelconque des revendications 1 à 3, l'excitation des variations dynamiques stimulées provoquant des propriétés de déformation du système optique, **caractérisé en ce que** ces variations des propriétés de déformation sont détectées de façon dynamique pendant une séquence temporelle à l'aide d'un aberromètre (1).

5. Dispositif de mesure du comportement dynamique d'un oeil humain ou artificiel, comprenant une unité de stimulation (4), pouvant exercer une excitation sur l'oeil, un aberromètre (1), l'aberromètre comprenant un dispositif d'analyse de front d'ondes (3), un dispositif d'acquisition dynamique de données (2) permuttant de recevoir des données brutes de l'aberromètre, **caractérisé par** la présence d'une unité de synchronisation (5) servant à la synchronisation entre l'unité de stimulation et l'aberromètre.
